# EUROPEAN PATENT SPECIFICATION

(11) **EP 4 505 409 B1**
(45) Date of publication and mention of the grant of the patent: **17.09.2025**
(21) Application number: 23713904.3
(22) Date of filing: 24.03.2023
(51) Int. Cl.: G06T 7/33, G06T 7/11, G06T 3/14, G06V 10/24

(54) **COMBINED RIB AND SPINE IMAGE PROCESSING FOR FAST ASSESSMENT OF SCANS**
KOMBINIERTE RIPPEN- UND WIRBELSÄULENBILDVERARBEITUNG ZUR SCHNELLEN BEURTEILUNG VON SCANS
TRAITEMENT COMBINÉ DES IMAGES DES CÔTES ET DE LA COLONNE VERTÉBRALE POUR UNE ÉVALUATION RAPIDE DES ACQUISITIONS

(30) Priority: 01.04.2022 US 202263326302 P; 07.07.2022 EP 22183627
(43) Date of publication of application: 12.02.2025
(73) Proprietor: Koninklijke Philips N.V., 5656 AG Eindhoven (NL)
(72) Inventor: BUERGER, Christian, 5656 AG Eindhoven (NL); LOSSAU, Tanja, 5656 AG Eindhoven (NL); KLINDER, Tobias, 5656 AG Eindhoven (NL); LORENZ, Cristian, 5656 AG Eindhoven (NL); GOSHEN, Liran, 5656AG Eindhoven (NL); SEGEV-STEINBUCH, Lior, 5656 AG Eindhoven (NL); KIMMEL, Yotam, 5656 AG Eindhoven (NL)
(74) Representative: Philips Intellectual Property & Standards
(86) International application number: PCT/EP2023/057631
(87) International publication number: WO 2023/186729

(56) References cited:
- EP-A1- 3 451 287
- H MARTINKE ET AL: "Bone Fracture and Lesion Assessment using Shape-Adaptive Unfolding", PROCEEDINGS OF EUROGRAPHICS WORKSHOP ON VISUAL COMPUTING FOR BIOLOGY AND MEDICINE, 7 September 2016 (2016-09-07), pages 149 - 158, XP055635792, Retrieved from the Internet <URL:https://diglib.eg.org/bitstream/handle/10.2312/vcbm20171249/149-158.pdf?sequence=1&isAllowed=y> [retrieved on 20221223], DOI: 10.2312/vcbm20171249
- HELMUT RINGL ET AL: "The ribs unfolded - a CT visualization algorithm for fast detection of rib fractures: effect on sensitivity and specificity in trauma patients", EUROPEAN RADIOLOGY, vol. 25, no. 7, 14 February 2015 (2015-02-14), Berlin/Heidelberg, pages 1865 - 1874, XP055225320, ISSN: 0938-7994, DOI: 10.1007/s00330-015-3598-2
- TOSUN D ET AL: "A Geometry-Driven Optical Flow Warping for Spatial Normalization of Cortical Surfaces", IEEE TRANSACTIONS ON MEDICAL IMAGING, IEEE, USA, vol. 27, no. 12, December 2008 (2008-12-01), pages 1739 - 1753, XP011227084, ISSN: 0278-0062, DOI: 10.1109/TMI.2008.925080

## Description

### FIELD OF THE INVENTION

The invention relates to the field of medical imaging and, in particular, to a method and apparatus for automatically generating a visualization of the ribs and spine.

### BACKGROUND OF THE INVENTION

Medical professionals commonly use medical imaging to visualize a patient's anatomy where the visualization data is used to diagnose diseases or injuries. In a trauma setting, medical personal may rely on medial image data in the form of a Computed Tomography (CT) scan for diagnostic purposes, for example, the detection of rib fractures.

Looking at thoracic or whole-body three-dimensional (3D) CT scans slice by slice is often a time-consuming process, especially when the target anatomical structure spans multiple slices (e.g., 24 individual ribs to trace).

Reading imaging scans and, more specifically, trauma or Emergency Department scans is a time-critical task that needs to be done with high attention to avoid overlooking critical findings. Often, in these settings, imaging is based on whole-body scans which results in large amounts of imaging data being generated that must be thoroughly inspected. The ribs and spine are especially critical structures to be assessed. These are repetitive structures which take a significant amount of time to examine, and in the case of the spine these structures are critical from a neurological perspective.

A visualization of the patient's anatomy and specific critical structures in an intuitive way could simplify the time consuming inspection that is currently required. Several approaches to simplify the visualization and evaluation of a patient's anatomy and critical structures have been proposed, especially those targeting visualization of the rib cages. While providing some benefit, current approaches come with distinct fundamental limitations.

One well-known visualization scheme is the "filet view" or "fishbone view." This view is based on segmenting the ribs (e.g., using a deep convolutional neural network) followed by a centerline extractor which subsequently labels rib pairs in the field of view. Each rib is sampled along its trace allowing a visualization of each and every rib in a normalized and straightened manner (curved planar reformat). This view allows medical professionals to accurately inspect the rib centerlines in a normalized view where all the ribs are straightened and placed to a unique position on the inspection canvas (reformatted view).

There are, however, several shortcomings with this type of view. One disadvantage with this type of view is that the nature of processing each rib independently leads to discontinuities between ribs, which may lead to imaging artifacts from adjacent ribs appearing in rib shapes. In addition, this view is limited to the ribs only, and adding context information such as the spine would require additional steps and imaging processing that would need to be done separately.

Another visualization scheme is the visceral cavity view. In this view, a segmentation algorithm (e.g., using a model-based approach) is applied to segment the inside of the rib cage in terms of a deformation of a cylindric manifold. Once segmentation is done, the manifold may be unwrapped and a maximum intensity projection (MIP) close to the proximity of the surface may be computed. This view allows the user to inspect the rib cage as a whole in terms of a continuous visualization on the inspection canvas.

One of the deficiencies with this type of view is that the relative rib lengths are not maintained. The nature of unwrapping a cylinder manifold does not allow visualization of the correct rib lengths. For example, the first rib appears too long in relation to the other ribs. In addition, the nature of the MIP visualization may not allow detection of subtle fractures. For example, the MIP visualization may make small rib fractures invisible and not detectable in the generated view. Another deficiency is that this view adds significant unrealistic distortions (wavy ribs) which limit clinical confidence in the generated visceral cavity view.

Thus, the need exists for an innovative visualization scheme that combines the advantages of the filet view and the visceral cavity view without the disadvantages associated with these views.

The article "Bone Fracture and Lesion Assessment using Shape-Adaptive Unfolding", by H. Martinke et al, Proceedings of Eurographics Workshop on Visual Computing for Biology and Medicine, 7 September 2016, pages 149-158, doi:10.2312/vcbm20171249, discloses a rib unfolding strategy that considers the cross-sectional shape of each rib individually and independently leading to shape-adaptive slices through the ribs, providing a concise overview visualization.

The article "The ribs unfolded - a CT visualization algorithm for fast detection of rib fractures: effect on sensitivity and specificity in trauma patients", by Helmut Ringl et al, European Radiology, vol. 25, no. 7, 14 February 2015, pages 1865-1874, ISSN 0938-7994, doi:10.1007/s00330-015-3598-2, compares the radiologist's detection rate of rib fractures in trauma CT when reading curved planar reformats of the ribs compared to reading standard MPRs.

The article "A Geometry-Drive Optical Flow Warping for Spatial Normalization of Cortical Surfaces", by Tosun D et al, IEEE Transactions on Medical Imaging, IEEE, USA, vol. 27, no. 12, December 2008, pages 1739-1753, ISSN 0278-0062, doi:10.1109/TMI.2008.925080, discloses a multichannel optical flow warping procedure to align two shape measures, that distinguish the sulcal and gyral regions in a multiscale framework, between a reference brain and a subject brain, creating the desired normalization.

Patent application EP3451287A1 discloses segmenting a body cavity in a 3D diagnostic image and determining (and visualizing) its boundary surface and the surface texture by projecting image information from a local neighborhood on the boundary surface.

### SUMMARY OF THE INVENTION

The object of the present invention provides techniques for generating a new manifold view that overcomes the deficiencies in existing visualization schemes. The techniques may be applied to a number of imaging systems including CT, CT-arm, Single Photon Emission Computer Tomography CT (SPECT-CT), Magnetic Resonance CT (MR-CT), Positron Emission Tomography CT (PET-CT), and Magnetic Resonance Imaging (MRI) systems.

According to a first aspect of the invention, an image processing device is provided. The image processing device comprises a memory configured to store computer executable instructions and at least one processor configured to execute the computer executable instructions to cause the image processing device to receive data representative of a three-dimensional diagnostic image, the three-dimensional diagnostic image including ribs and spine of a subject. The computer executable instructions cause the image processing device to segment the ribs and spine, according to the received data representative of the three-dimensional diagnostic image, detect and label rib centerlines from the rib segmentation, detect and label vertebra body center landmarks from the spine segmentation, and define a two-dimensional manifold plane to signify a visualization canvas for displaying a reformatted image. The computer executable instructions cause the image processing device to map each three-dimensional position, corresponding to the received data representative of the three-dimensional diagnostic image, of the rib centerlines and the vertebra center landmarks to a two-dimensional position on the defined two-dimensional plane, interpolate three-dimensional position coordinates missing on the defined two-dimensional manifold plane, which deforms the two-dimensional manifold such that it aligns with the detected rib centerlines and vertebra center landmarks, sample image intensities, from three-dimensional diagnostic image space, at each coordinate of the deformed two-dimensional manifold plane. A reformatted image is generated as a manifold slice from the sampled image intensities, the manifold slice displaying a continuous and straightened visualization of a rib cage and spine. The deformed two-dimensional manifold plane is shifted along its normal directions and the sampling is repeated to generate a stack of manifold slices covering a complete three-dimensional visualization of the rib cage. A three-dimensional visualization of the rib cage and the vertebra body centers is generated as a stack of manifolds covering the complete rib cage.

In a second aspect of the invention, a method of processing a three-dimensional image is provided. The method comprises receiving data representative of a three-dimensional diagnostic image, the three-dimensional diagnostic image including ribs and spine of a subject, segmenting the ribs and spine according to the received data representative of the three dimensional diagnostic image, detecting and labeling rib centerlines from the rib segmentation, detecting and labeling vertebra body center landmarks from the spine segmentation, and defining a two-dimensional manifold plane to signify a visualization canvas for displaying a reformatted image. The method comprises mapping each three-dimensional position, corresponding to the received data representative of the three-dimensional diagnostic image, of the rib centerlines and the vertebra center landmarks to a two-dimensional position on the defined two-dimensional plane, interpolating three-dimensional position coordinates missing on the defined two dimensional manifold plane, which deforms the two-dimensional manifold such that it aligns with the detected rib centerlines and vertebra center landmarks, and sampling image intensities, from three-dimensional diagnostic image space, at each coordinate of the deformed two-dimensional manifold plane. A reformatted image is generated as a manifold slice from the sampled image intensities, the manifold slice displaying a continuous and straightened visualization of a rib cage and spine. The deformed two-dimensional manifold plane is shifted along its normal directions, and the sampling is repeated to generate a stack of manifold slices covering a complete three-dimensional visualization of the rib cage. A three-dimensional visualization of the rib cage and the vertebra body centers is generated as a stack of manifolds covering the complete rib cage.

In a third aspect of the invention, a non-transitory computer-readable medium having stored thereon instructions for causing processing circuitry to execute a process is provided. The process comprises receiving data representative of a three-dimensional diagnostic image, the three-dimensional diagnostic image including ribs and spine of a subject, segmenting the ribs and spine, according to the received data representative of the three-dimensional diagnostic image, detecting and labeling rib centerlines from the rib segmentation, detecting and labeling vertebra body center landmarks from the spine segmentation, and defining a two-dimensional manifold plane to signify a visualization canvas for displaying a reformatted image, the two-dimensional manifold plane being deformed to align with the detected rib centerlines and vertebra center landmarks. The process further includes mapping each three-dimensional position, corresponding to the received data representative of the three-dimensional diagnostic image, of the rib centerlines and the vertebra center landmarks to a two-dimensional position on the defined two-dimensional plane, interpolating three-dimensional position coordinates missing on the defined two dimensional manifold plane, which deforms the two-dimensional manifold such that it aligns with the detected rib centerlines and vertebra center landmarks. Image intensities, from three-dimensional diagnostic image space, are sampled at each coordinate of the deformed two-dimensional manifold plane. A reformatted image is generated as a manifold slice from the sampled image intensities, the manifold slice displaying a continuous and straightened visualization of a rib cage and spine. The deformed two-dimensional manifold plane is shifted along its normal directions, and the sampling is repeated to generate a stack of manifold slices covering a complete three-dimensional visualization of the rib cage. A three-dimensional visualization of the rib cage and the vertebra body centers is generated as a stack of manifolds covering the complete rib cage.

In a preferred embodiment, segmenting the ribs and the spine is performed with machine learning or deep learning techniques. The machine learning or deep learning techniques including at least one of neural networks, logistic regression, random forests, nearest neighbors, and cluster or multivariate analysis. The segmentation approach using deep learning is applied to segment the ribs and spine followed by a rib centerline extraction, as well as vertebra body center landmark detections. As a result, all rib pairs and vertebra body centers of gravity are accurately detected and labelled in an automated manner.

A two-dimensional manifold plane, which will be used to represent the visualization canvas for displaying a reformatted image, is defined. Each three-dimensional position of the detected rib centerlines and vertebra center landmarks is mapped to a two-dimensional position on the visualization canvas which will visualize the unfolded ribcage. In other words, coordinate correspondences from two-dimensional space to three-dimensional space are defined.

Once the two-dimensional to three-dimensional coordinates are established, the correspondences from the remaining pixels in two-dimensional space are still lagging. In an aspect of the invention, an interpolation scheme is implemented that interpolates the x/y/z coordinate correspondences from three-dimensional space to two-dimensional space in such regions where this information is not yet available.

In a preferred embodiment, the interpolation is performed with thin plate spline techniques. Thin plate splines are a good choice for an interpolation technique as it models originally a deformation of a thin two-dimensional metal plate. Image intensities from the three-dimensional diagnostic image space are sampled at each coordinate of the deformed two-dimensional manifold plane. Thus, a reformatted image is generated as a manifold slice from the sampled image intensities, the manifold slice displaying a continuous and straightened visualization of a rib cage and spine.

In an aspect of the invention, the sampled image intensities at each position of the deformed two-dimensional manifold plane correspond to at least one of a region of tissue between the ribs and a region of tissue bordering the ribs.

In an aspect of the invention, a stack of interpolated two-dimensional multiplanar reconstructions (MPRs) is generated by computing the normal directions for each point on the two-dimensional plane and shifting or moving the manifold surface multiple times toward the inside and outside of the manifold plane. A three-dimensional image of the rib cage and spine is then generated based on the stack of two-dimensional MPRs.

### BRIEF DESCRIPTION OF THE DRAWINGS

In the following drawings:
Fig. 1 is a diagram of an exemplary imaging system;
Fig. 2 illustrates a view of a rib cage according to the "filet view" or "fishbone view";
Fig. 3 illustrates a visceral cavity view of a rib cage;
Fig. 4 is a flowchart representing a method generating a combined rib and spine view according to some embodiments;
Fig. 5 illustrates mapping from three-dimensional space to two-dimensional space according to some embodiments;
Fig. 6. illustrates a processing workflow according to some embodiments; and
Fig. 7 illustrates a 3D view of the ribs and spine according to some embodiments.

### DETAILED DESCRIPTION OF EMBODIMENTS

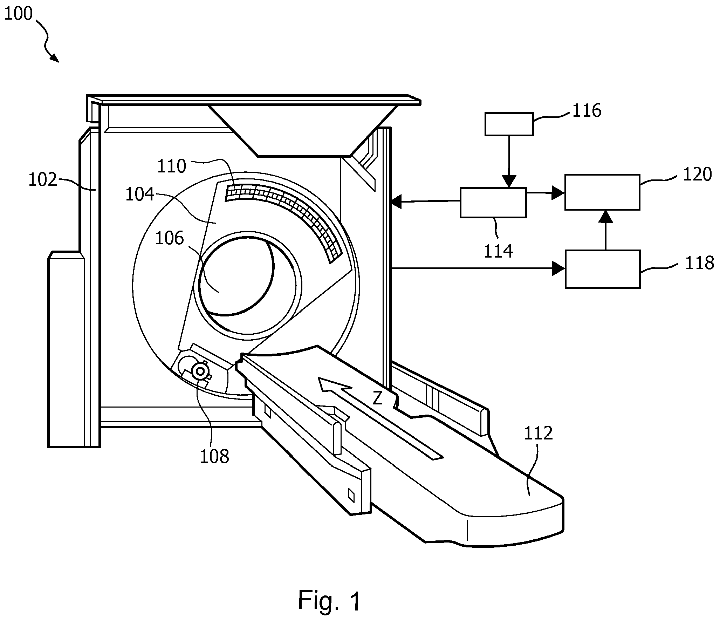

Fig. 1 illustrates an imaging system 100, such as a computed tomography (CT) imaging system. The imaging system 100 includes a generally stationary gantry 102 and a rotating gantry 104. The rotating gantry 104 is rotatably supported by the stationary gantry 102 and rotates in relation to the stationary gantry 102 around an examination region 106 about a longitudinal or z-axis.

A patient support 112, such as a couch, supports an object or examination subject, such as a human patient, in the examination region 106. The support 112 is configured to move the object or examination subject for loading, scanning, and unloading.

A radiation source 108, such as an x-ray tube, is rotatably supported by the rotating gantry 104. The radiation source 108 rotates with the rotating gantry 104 and emits radiation that traverses the examination region 106.

A radiation sensitive detector array 110 subtends an angular arc opposite the radiation source 108 across the examination region 106. The detector array 110 includes one or more rows of detectors that extend along the z-axis direction, detects radiation traversing the examination region 106, and generates projection data indicative thereof.

A general-purpose computing system or computer serves as an operator console 114 and includes an input device(s) 116, such as a mouse, a keyboard, and/or the like, and an output device(s) 120, such as a display monitor, a filmer or the like. The console 114 allows an operator to control the operation of the system 100. This includes control of an image processing device 118. The image processing device 118 may receive data representative of a three-dimensional (3D) diagnostic image generated by the imaging system 100 and generate a reformatted image displaying a continuous and straightened visualization of a rib cage and spine. The reformatted image may be output to output device 120 for reading by medical personnel.

It is to be appreciated that processing of the image processing device 118 can be implemented through a processor(s) and/or processing circuitry, which execute a computer readable instruction(s), such as executable code, encoded or embedded on computer readable storage medium such as physical memory and other non-transitory medium. Additionally or alternatively, the processor(s) and/or processing circuitry can execute a computer readable instruction(s) carried by a carrier wave, a signal and other transitory (or non, non-transitory) medium.

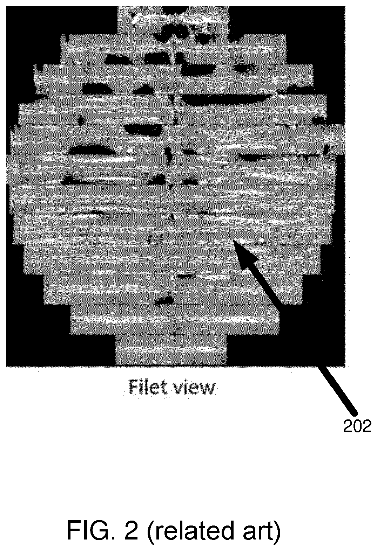

Fig. 2 illustrates a view of a rib cage according to the "filet view" or "fishbone view" visualization scheme. It can be seen that this view allows a medical professional to accurately inspect the rib centerlines in a normalized view (reformatted view), where all the ribs are straightened and placed to a unique position on the inspection canvas.

One disadvantage with this view is that the nature of processing each rib independently leads to discontinuities between ribs, which may lead to imaging artifacts appearing in rib shapes from adjacent ribs as identified at 202. Another disadvantage is that the view is limited to the ribs only.

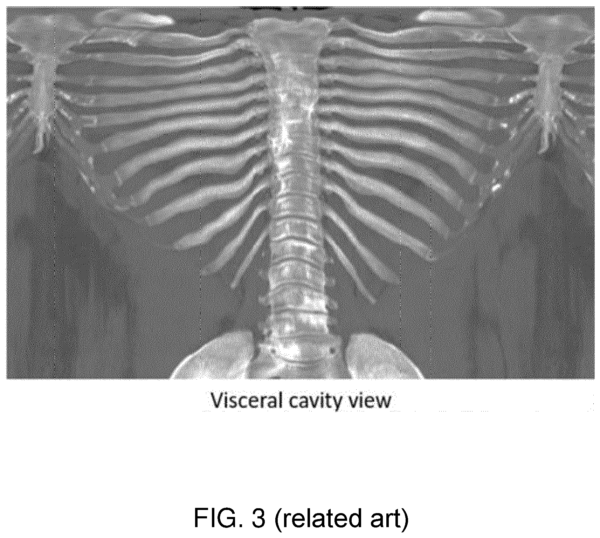

Fig. 3 illustrates a visceral cavity view. In this visual scheme, a segmentation algorithm using a model-based approach, for example, is applied to segment the inside of the rib cage in terms of deformation of a cylindric manifold. Once segmentation is done, the manifold can be unwrapped and a maximum intensity projection (MIP) close to the proximity of the surface can be computed.

This view allows the user to inspect the rib cage as a whole in terms of a continuous visualization on the inspection canvas. One of the deficiencies with the visceral cavity view that can be seen in Fig. 3 is that the relative rib lengths are not maintained. For example, the first rib appears too long in relation to the other ribs. In addition, the nature of the MIP visualization may not allow detection of subtle fractures. For example, the MIP visualization may make small rib fractures invisible and not detectable in the generated view. Another deficiency is that this view adds significant unrealistic distortions (wavy ribs), which limit clinical confidence in the generated visceral cavity view.

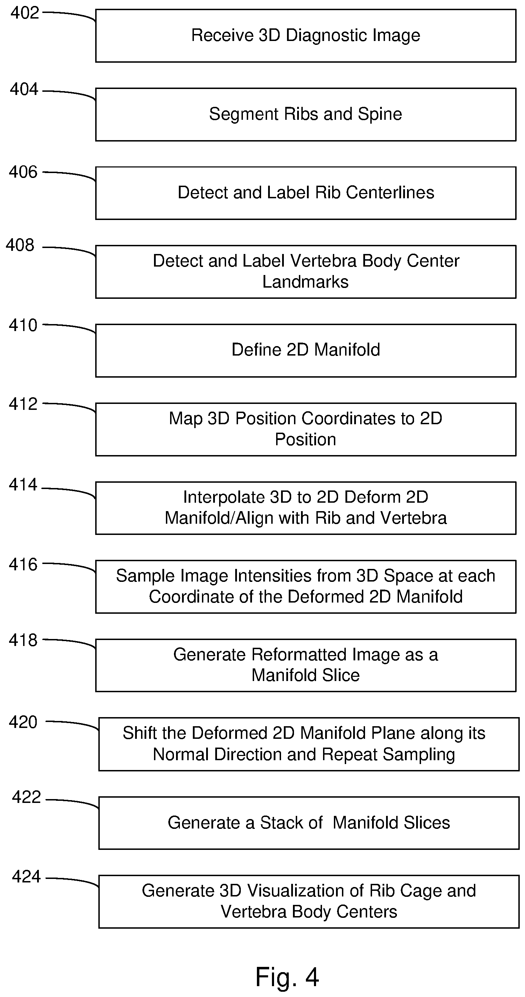

Fig. 4 is a flowchart representing a method of generating a reformatted medical image. The type of medical imaging performed may be, for example, a CT scan producing a three-dimensional CT image of a human patient.

At block 402, a three-dimensional diagnostic image is received. As an example, the three-dimensional diagnostic image may be a CT imaging system scan. At block 404, the ribs and spine of the scanned patient are automatically segmented. Segmenting may be performed using machine learning or deep learning techniques. As an example, Convolutional Neural Networks (CNNs) are a deep learning technique that may be used to segment the ribs and spine. CNN architectures, such as U-Net and V-Net, may be adapted for a segmentation task. Deep learning based training, using datasets from hundreds of scans, may be used to develop algorithms that are shown to achieve fast and accurate segmentation of the ribs and spine.

As part of the process, at block 406, the ribs are detected and the rib centerlines are extracted and labeled, and at block 408, vertebra body center landmarks are detected and labeled. At block 410, a two-dimensional (2D) manifold plane is defined. The 2D manifold plane is defined to represent a visualization canvas for a reformatted image that will be generated later. The 2D manifold is defined such that the visualization canvas for a reformatted image is a standardized visualization of the rib cage. Thus, the standardized visualization of the rib cage may be used for comparing with follow-up scans without the need for image registration, as the standardized views will be already registered. In other words, one-to-one mapping between coordinates in one space and those in another space is determined such that points in the two spaces correspond to the same anatomical point.

At block 412, each three-dimensional (3D) position of the rib centerlines and the vertebra center landmarks corresponding to the scan image data, the received data representative of the three-dimensional diagnostic image, is mapped to a 2D position on the defined two-dimensional manifold plane. Since the rib and vertebra positions are known in 3D space, correlations for all rib centerlines and all vertebra landmarks can be built up. That is, coordinate correspondences from 3D space to 2D space may be defined as a mapping function. Once the 3D to 2D correspondences are established or setup, correspondences or correlations from the remaining pixels in the defined 2D space may be missing.

Thus, at block 414, an interpolation scheme is implemented to interpolate the x/y/z coordinate correspondences from the 3D space to the defined 2D space in regions where this information is missing. This information may include the region of tissue between the ribs and the region of tissue bordering the ribs. This information may even include parts of the lungs which might be extremely relevant in the case of co-existing of pneumothorax as well as hemothorax. The interpolation scheme deforms the defined 2D manifold such that it aligns with the detected rib centerlines and vertebra center landmarks. Thin plate splines are a good choice for use as an interpolation scheme as it originally models a deformation of a thin 2D metal plate. Elastic body splines (EBS) is another approach to mapping or coordinate transformation that is suitable for medical image analysis.

At block 416, image intensities from the 3D diagnostic image space are sampled at each coordinate of the deformed 2D manifold plane. Sampling the image intensities from the 3D diagnostic image space at each coordinate of the deformed 2D manifold plane finally unwraps the rib cage and leads to the reformatted image. Sampling the image intensities may be based on the coordinate correspondences from 3D space to 2D space, as a mapping function. That is, the sampling is performed according to a coordinate mapping function corresponding to the mapped and interpolated positions.

At block 418, the reformatted image is generated as a manifold slice from the sampled image intensities. The reformatted image generated as a manifold slice displays a continuous and straightened visualization of a rib cage and spine. The reformatted image combines the advantages of both the filet and visceral cavity view displaying normalized and straightened rib views while maintaining relative rib lengths, and the reformatted image avoids the disadvantages of both the filet and visceral cavity view including discontinuities between the ribs, imaging artifacts appearing from adjacent rib positions, incorrect rib length, and invisible fractures and distortions due to MIP. The reformatted image that is generated at block 418 may be output to medical professionals for diagnostic purposes.

To facilitate further inspection and diagnoses, a stack of 2D manifold slices may be generated. At block 420, the 2D manifold plane is shifted along its normal directions toward the inside and outside of the manifold plane and the sampling is repeated for each incremental shift along the normal direction to generate a stack of manifold slices at block 422. The stack of manifold slices covers a complete 3D visualization of the rib cage and spine.

At block 424, a 3D visualization covering the complete rib cage and vertebra centers is generated as a stack of manifolds covering the complete rib cage. The stack of manifold slices generated at block 422 may be a stack of interpolated 2D multiplanar reconstructions (MPRs), and a 3D image of the rib cage and spine may be generated at block 424 based on the stack of 2D MPRs. The generated 3D visualization covering the complete rib cage and vertebra centers allows inclusion of all the ribs a whole. This allows medical professionals to easily browse through the rib cage for a quick visual inspection of the rib cage and to look for rib and spine fractures.

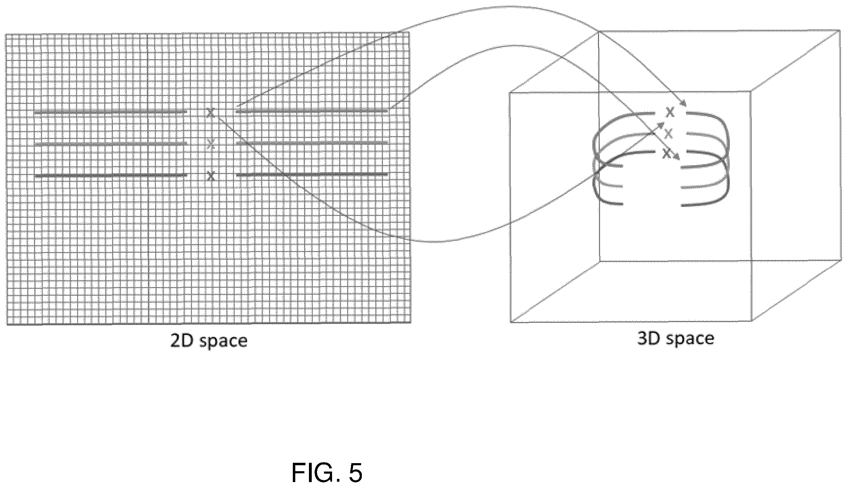

Fig. 5 illustrates mapping from 3D space to 2D space. Positions of the rib and vertebra are known in the 3D space. Each position of the rib centerlines and the vertebra center landmarks in 3D space are mapped to a corresponding position of a defined 2D manifold plane. The defined 2D manifold plane being a visualization canvas for the 2D manifold image that will be generated subsequently. Essentially, the ribs are being traced and mapped to pixel positions where each rib centerline and the vertebra body center landmarks will appear in the 2D manifold. For ease of description, only three ribs and vertebra body center landmarks are illustrated in Fig. 5. It should be understood, however, that all ribs and vertebra body center landmarks corresponding to the rib and vertebra body center landmarks that are known in the 3D space are mapped to positions in the defined 2D manifold plane.

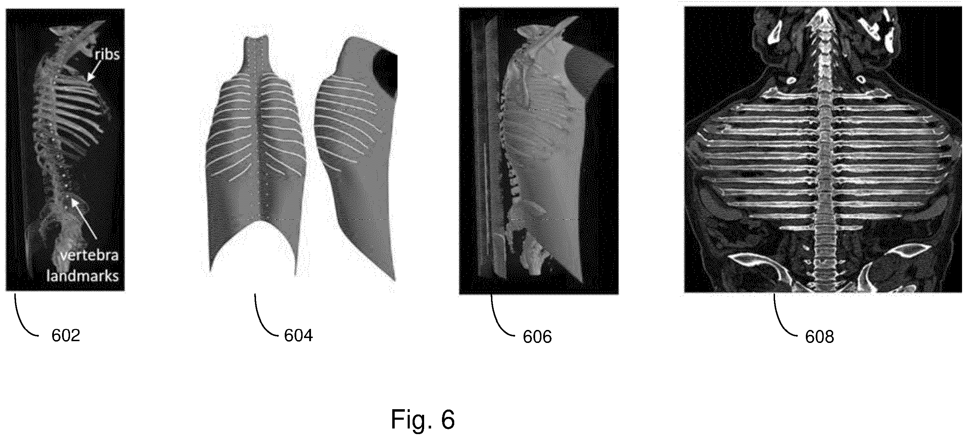

Fig. 6 illustrates a processing workflow according to some embodiments. At 602, rib centerlines and vertebra body center landmarks are detected and labeled according to an automated segmentation scheme. The image segmentation scheme may be based on machine learning or deep learning techniques. A two-dimensional manifold, which will show the resulting reformatted view, is defined. At 604 an interpolation scheme deforms the two-dimensional manifold such that it aligns with the detected rib centerlines and vertebra center landmarks. Thin plate splines or any appropriate interpolation scheme may be used. As illustrated in 604, a defined 2D manifold is deformed to align with the detected rib centerlines and vertebra landmarks mapped from 3D space to the 2D space of the 2D deformed manifold.

Image intensities from the 3D image space, for example a 3D image from a CT scan, are sampled at each coordinate of the deformed 2D manifold to complete or fill in the missing 3D coordinates as illustrated at 606. The missing 3D coordinates may correspond to a region of tissue between the ribs and a region of tissue bordering the ribs or any combination of a region of tissue between the ribs and a region of tissue bordering the ribs. This allows the deformed 2D manifold to be unfolded.

The unfolded 2D manifold is illustrated at 608. The new manifold view, illustrated at 608 as a reformatted image, combines the advantages of both the filet and visceral cavity views but avoids the disadvantages of both the filet and visceral cavity views. It can be seen at 608 that a normalized and straightened view of the ribs is displayed while maintaining the relative rib lengths. Avoided are discontinuities between the ribs, imaging artifacts appearing from adjacent rib positions, incorrect rib length, and invisible fractures and distortions due to MIP.

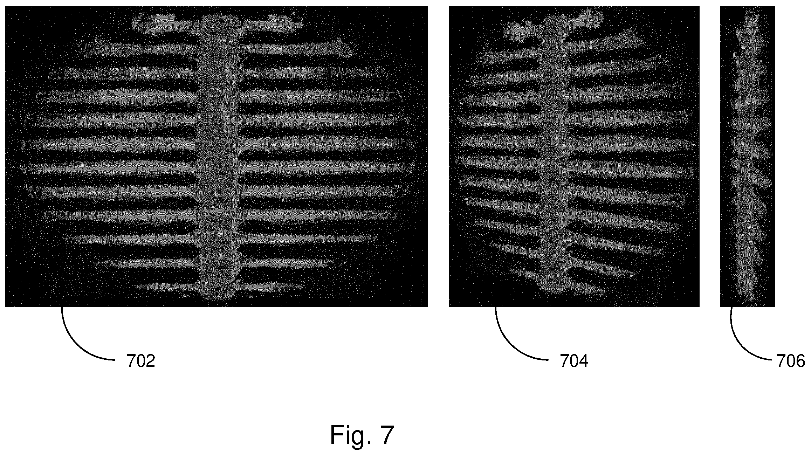

Fig. 7 illustrates a 3D view of the ribs and spine according to some embodiments. A 3D visualization of the rib cage and vertebra body centers may be generated by shifting or moving the deformed 2D manifold plane along its normal directions toward the inside and outside of the manifold plane multiple times and repeating the sampling to generate a stack of 2D manifold slices. This results in a 3D image as a stack of manifolds covering the complete rib cage. Fig. 7 illustrates that the resulting 3D image allows inclusion of all ribs as a whole.

The stack or manifold slices may be a stack or interpolated two-dimensional multiplanar reconstructions (MPRs), and a 3D image of the ribcage and spine may be generated based on the stack of 2D MPRs. The generated 3D rendering is shown at 702. The view at 704 is rotated about thirty degrees, and the view at 706 is rotated slightly under ninety degrees.

While the invention has been illustrated and described in detail in the drawings and foregoing description, such illustrations and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. The scope of the invention is given by the appended claims.

In the claims, the word "comprising" does not exclude other elements or steps, and the indefinite article "a" or "an" does not exclude a plurality.

A single processor, device or other unit may fulfill the functions of several items recited in the claims. The mere fact that certain measures are recited in mutually different dependent claims does not indicate that a combination of these measures cannot be used to advantage.

Operations like acquiring, determining, obtaining, outputting, providing, store or storing, calculating, simulating, receiving, warning, and stopping can be implemented as program code means of a computer program and/or as dedicated hardware.

A computer program may be stored and/or distributed on a suitable medium, such as an optical storage medium or a solid-state medium, supplied together with or as part of other hardware, but may also be distributed in other forms, such as via the Internet or other wired or wireless telecommunication systems.

## Claims

1. An image processing device, comprising:
a memory configured to store computer executable instructions; and
at least one processor configured to execute the computer executable instructions to cause the image processing device to:
receive (402) data representative of a three-dimensional diagnostic image, the three-dimensional diagnostic image including ribs and spine of a subject;
segment (404) the ribs and spine according to the received data representative of the three-dimensional diagnostic image;
detect and label (406) rib centerlines from the rib segmentation;
detect and label (408) vertebra body center landmarks from the spine segmentation;
define (410) a two-dimensional manifold plane to signify a visualization canvas for displaying a reformatted image;
map (412) each three-dimensional position, corresponding to the received data representative of the three-dimensional diagnostic image, of the rib centerlines and the vertebra center landmarks to a two-dimensional position on the defined two-dimensional plane;
interpolate (414) three-dimensional position coordinates missing on the defined two-dimensional manifold plane, which deforms the two-dimensional manifold such that the two-dimensional manifold aligns with the detected rib centerlines and vertebra center landmarks;
sample (416) image intensities, from three-dimensional diagnostic image space, at each coordinate of the deformed two-dimensional manifold plane;
generate (418) the reformatted image as a manifold slice from the sampled image intensities, the manifold slice displaying a continuous and straightened visualization of a rib cage and spine;
shift (420) the deformed two-dimensional manifold plane along its normal directions and repeat the sampling to generate (422) a stack of manifold slices covering a complete three-dimensional visualization of the rib cage and spine; and
generate (424) a three-dimensional visualization of the rib cage and the vertebra body centers as a stack of manifolds covering the complete rib cage.

2. The image processing device according to claim 1, wherein segmenting the ribs and the spine is performed with machine learning or deep learning techniques, the machine learning or deep learning techniques including at least one of neural networks, logistic regression, random forests, nearest neighbors, and cluster or multivariate analysis.

3. The image processing device according to claim 1, wherein the interpolating is performed with thin plate spline techniques.

4. The image processing device according to claim 1, wherein the processor is further configured to execute the computer executable instructions to cause the image processing device to compute the normal directions for each point on the two-dimensional plane.

5. The image processing device according to claim 1, wherein the generated stack of manifold slices is a stack of interpolated two-dimensional multiplanar reconstructions.

6. The image processing device according to claim 5, wherein the processor is further configured to execute the computer executable instructions to generate a three-dimensional image of the rib cage and spine based on the stack of two-dimensional multiplanar reconstructions.

7. The image processing device according to claim 1, wherein the sampled image intensities at each position of the deformed two-dimensional manifold plane correspond to at least one of a region of tissue between the ribs and a region of tissue bordering the ribs.

8. A computer-implemented method of processing a three-dimensional image, the method comprising
receiving (402) data representative of a three-dimensional diagnostic image, the three-dimensional diagnostic image including ribs and spine of a subject;
segmenting (404) the ribs and spine according to the received data representative of the three dimensional diagnostic image;
detecting and labeling (406) rib centerlines from the rib segmentation;
detecting and labeling (408) vertebra body center landmarks from the spine segmentation;
defining (410) a two-dimensional manifold plane to signify a visualization canvas for displaying a reformatted image;
mapping (412) each three-dimensional position, corresponding to the received data representative of the three-dimensional diagnostic image, of the rib centerlines and the vertebra center landmarks to a two-dimensional position on the defined two-dimensional plane;
interpolating (414) three-dimensional position coordinates missing on the defined two dimensional manifold plane, which deforms the two-dimensional manifold such that the two-dimensional manifold aligns with the detected rib centerlines and vertebra center landmarks;
sampling (416) image intensities, from three-dimensional diagnostic image space, at each coordinate of the deformed two-dimensional manifold plane;
generating (418) the reformatted image as a manifold slice from the sampled image intensities, the manifold slice displaying a continuous and straightened visualization of a rib cage and spine;
shifting (420) the deformed two-dimensional manifold plane along its normal directions and repeating the sampling to generate (422) a stack of manifold slices covering a complete three-dimensional visualization of the rib cage and spine; and
generating (424) a three-dimensional visualization of the rib cage and the vertebra body centers as a stack of manifolds covering the complete rib cage.

9. The method according to claim 8, wherein the segmenting is performed with machine learning or deep learning techniques, the machine learning or deep learning techniques including at least one of neural networks, logistic regression, random forests, nearest neighbors, and cluster or multivariate analysis.

10. The method according to claim 8, wherein the interpolating is performed with thin plate spline techniques.

11. The method according to claim 8, further comprising computing the normal directions for each point on the two-dimensional plane.

12. The method according to claim 8, wherein the generated stack of manifold slices is a stack of interpolated two-dimensional multiplanar reconstructions.

13. The method according to claim 12, further comprising generating a three-dimensional image of the rib cage and spine based on the stack of interpolated two-dimensional multiplanar reconstructions.

14. The method according to claim 8, wherein the sampled image intensities at each position of the deformed two-dimensional manifold plane correspond to at least one of a region of tissue between the ribs and a region of tissue bordering the ribs.

15. A non-transitory computer-readable medium having stored thereon instructions for causing processing circuitry to execute a process, the process comprising:
receiving data representative of a three-dimensional diagnostic image, the three-dimensional diagnostic image including ribs and spine of a subject;
segmenting the ribs and spine according to the received data representative of the three-dimensional diagnostic image;
detecting and labeling rib centerlines from the rib segmentation;
detecting and labeling vertebra body center landmarks from the spine segmentation;
defining a two-dimensional manifold plane to signify a visualization canvas for displaying a reformatted image, the two-dimensional manifold plane being deformed to align with the detected rib centerlines and vertebra center landmarks;
mapping each three-dimensional position, corresponding to the received data representative of the three-dimensional diagnostic image, of the rib centerlines and the vertebra center landmarks to a two-dimensional position on the defined two-dimensional plane;
interpolating three-dimensional position coordinates missing on the defined two dimensional manifold plane, which deforms the two-dimensional manifold such that the two-dimensional manifold aligns with the detected rib centerlines and vertebra center landmarks;
sampling image intensities, from three-dimensional diagnostic image space, at each coordinate of the deformed two-dimensional manifold plane;
generating the reformatted image as a manifold slice from the sampled image intensities, the manifold slice displaying a continuous and straightened visualization of a rib cage and spine;
shifting the deformed two-dimensional manifold plane along its normal directions and repeating the sampling to generate a stack of manifold slices covering a complete three-dimensional visualization of the rib cage; and
generating a three-dimensional visualization of the rib cage and the vertebra body centers as a stack of manifolds covering the complete rib cage.

## Patentansprüche

1. Bildverarbeitungsvorrichtung, umfassend:
einen Speicher, der zum Speichern von computerausführbaren Anweisungen konfiguriert ist; und
mindestens einen Prozessor, der zum Ausführen der computerausführbaren Anweisungen konfiguriert ist, um die Bildverarbeitungsvorrichtung zu veranlassen zum:
Empfangen (402) von Daten, die für ein dreidimensionales Diagnosebild repräsentativ sind, wobei das dreidimensionale Diagnosebild Rippen und Wirbelsäule eines Subjekts einschließt;
Segmentieren (404) der Rippen und Wirbelsäule gemäß den empfangenen Daten, die für das dreidimensionale Diagnosebild repräsentativ sind;
Erkennen und Beschriften (406) von Rippenmittellinien aus der Rippensegmentierung;
Erkennen und Beschriften (408) von Wirbelkörpermittelpunkt-Orientierungspunkten aus der Wirbelsäulensegmentierung;
Definieren (410) einer zweidimensionalen Mannigfaltigkeitsebene zur Bezeichnung einer Visualisierungsfläche zum Anzeigen eines neu formatierten Bildes;
Abbilden (412) jeder dreidimensionalen Position, die den empfangenen Daten entspricht, die für das dreidimensionale Diagnosebild repräsentativ sind, der Rippenmittellinien und der Wirbelmittelpunkt-Orientierungspunkte auf eine zweidimensionale Position auf der definierten zweidimensionalen Ebene;
Interpolieren (414) dreidimensionaler Positionskoordinaten, die auf der definierten zweidimensionalen Mannigfaltigkeitsebene fehlen, wodurch die zweidimensionale Mannigfaltigkeit so verformt wird, dass die zweidimensionale Mannigfaltigkeit mit den erkannten Rippenmittellinien und Wirbelmittelpunkt-Orientierungspunkten ausgerichtet ist;
Abtasten (416) von Bildintensitäten aus einem dreidimensionalen Diagnosebildraum an jeder Koordinate der verformten zweidimensionalen Mannigfaltigkeitsebene;
Generieren (418) des neu formatierten Bildes als Mannigfaltigkeitsschnitt aus den abgetasteten Bildintensitäten, wobei der Mannigfaltigkeitsschnitt eine kontinuierliche und begradigte Visualisierung eines Brustkorbs und einer Wirbelsäule anzeigt;
Verschieben (420) der verformten zweidimensionalen Mannigfaltigkeitsebene entlang ihrer Normalenrichtungen und Wiederholen der Abtastung, um einen Stapel von Mannigfaltigkeitsschnitten zu generieren (422), die eine vollständige dreidimensionale Visualisierung des Brustkorbs und der Wirbelsäule abdecken; und
Generieren (424) einer dreidimensionalen Visualisierung des Brustkorbs und der Wirbelkörpermittelpunkte als einen Stapel von Mannigfaltigkeiten, die den gesamten Brustkorb abdecken.

2. Bildverarbeitungsvorrichtung nach Anspruch 1, wobei Segmentieren der Rippen und der Wirbelsäule mit Techniken des maschinellen Lernens oder des Deep Learning durchgeführt wird, wobei die Techniken des maschinellen Lernens oder des Deep Learning mindestens eines von neuronalen Netzwerken, logistischer Regression, Zufallswäldern, nächsten Nachbarn und Cluster- oder multivariater Analyse einschließen.

3. Bildverarbeitungsvorrichtung nach Anspruch 1, wobei das Interpolieren mit Thin-Plate-Spline-Techniken durchgeführt wird.

4. Bildverarbeitungsvorrichtung nach Anspruch 1, wobei der Prozessor weiter dazu konfiguriert ist, die computerausführbaren Anweisungen auszuführen, um die Bildverarbeitungsvorrichtung zu veranlassen, die Normalenrichtungen für jeden Punkt auf der zweidimensionalen Ebene zu berechnen.

5. Bildverarbeitungsvorrichtung nach Anspruch 1, wobei der generierte Stapel aus Mannigfaltigkeitsschnitten ein Stapel aus interpolierten zweidimensionalen multiplanaren Rekonstruktionen ist.

6. Bildverarbeitungsvorrichtung nach Anspruch 5, wobei der Prozessor weiter dazu konfiguriert ist, die computerausführbaren Anweisungen auszuführen, um auf der Grundlage des Stapels zweidimensionaler multiplanarer Rekonstruktionen ein dreidimensionales Bild des Brustkorbs und der Wirbelsäule zu generieren.

7. Bildverarbeitungsvorrichtung nach Anspruch 1, wobei die abgetasteten Bildintensitäten an jeder Position der verformten zweidimensionalen Mannigfaltigkeitsebene mindestens einem von einem Gewebebereich zwischen den Rippen und einem an die Rippen angrenzenden Gewebebereich entsprechen.

8. Computerimplementiertes Verfahren zum Verarbeiten eines dreidimensionalen Bildes, wobei das Verfahren Folgendes umfasst:
Empfangen (402) von Daten, die für ein dreidimensionales Diagnosebild repräsentativ sind, wobei das dreidimensionale Diagnosebild Rippen und Wirbelsäule eines Subjekts einschließt;
Segmentieren (404) der Rippen und Wirbelsäule gemäß den empfangenen Daten, die für das dreidimensionale Diagnosebild repräsentativ sind;
Erkennen und Beschriften (406) von Rippenmittellinien aus der Rippensegmentierung;
Erkennen und Beschriften (408) von Wirbelkörpermittelpunkt-Orientierungspunkten aus der Wirbelsäulensegmentierung;
Definieren (410) einer zweidimensionalen Mannigfaltigkeitsebene zur Bezeichnung einer Visualisierungsfläche zum Anzeigen eines neu formatierten Bildes;
Abbilden (412) jeder dreidimensionalen Position, die den empfangenen Daten entspricht, die für das dreidimensionale Diagnosebild repräsentativ sind, der Rippenmittellinien und der Wirbelmittelpunkt-Orientierungspunkte auf eine zweidimensionale Position auf der definierten zweidimensionalen Ebene;
Interpolieren (414) dreidimensionaler Positionskoordinaten, die auf der definierten zweidimensionalen Mannigfaltigkeitsebene fehlen, wodurch die zweidimensionale Mannigfaltigkeit so verformt wird, dass die zweidimensionale Mannigfaltigkeit mit den erkannten Rippenmittellinien und Wirbelmittelpunkt-Orientierungspunkten ausgerichtet ist;
Abtasten (416) von Bildintensitäten aus einem dreidimensionalen Diagnosebildraum an jeder Koordinate der verformten zweidimensionalen Mannigfaltigkeitsebene;
Generieren (418) des neu formatierten Bildes als Mannigfaltigkeitsschnitt aus den abgetasteten Bildintensitäten, wobei der Mannigfaltigkeitsschnitt eine kontinuierliche und begradigte Visualisierung eines Brustkorbs und einer Wirbelsäule anzeigt;
Verschieben (420) der verformten zweidimensionalen Mannigfaltigkeitsebene entlang ihrer Normalenrichtungen und Wiederholen der Abtastung, um einen Stapel von Mannigfaltigkeitsschnitten zu generieren (422), die eine vollständige dreidimensionale Visualisierung des Brustkorbs und der Wirbelsäule abdecken; und
Generieren (424) einer dreidimensionalen Visualisierung des Brustkorbs und der Wirbelkörpermittelpunkte als einen Stapel von Mannigfaltigkeiten, die den gesamten Brustkorb abdecken.

9. Verfahren nach Anspruch 8, wobei Segmentieren mit Techniken des maschinellen Lernens oder des Deep Learning durchgeführt wird, wobei die Techniken des maschinellen Lernens oder des Deep Learning mindestens eines von neuronalen Netzwerken, logistischer Regression, Zufallswäldern, nächsten Nachbarn und Cluster- oder multivariater Analyse einschließen.

10. Verfahren nach Anspruch 8, wobei das Interpolieren mit Thin-Plate-Spline-Techniken durchgeführt wird.

11. Verfahren nach Anspruch 8, das weiter Berechnen der Normalenrichtungen für jeden Punkt auf der zweidimensionalen Ebene umfasst.

12. Verfahren nach Anspruch 8, wobei der generierte Stapel aus Mannigfaltigkeitsschnitten ein Stapel aus interpolierten zweidimensionalen multiplanaren Rekonstruktionen ist.

13. Verfahren nach Anspruch 12, das weiter Generieren eines dreidimensionalen Bildes des Brustkorbs und der Wirbelsäule auf der Grundlage des Stapels interpolierter zweidimensionaler multiplanarer Rekonstruktionen umfasst.

14. Verfahren nach Anspruch 8, wobei die abgetasteten Bildintensitäten an jeder Position der verformten zweidimensionalen Mannigfaltigkeitsebene mindestens einem von einem Gewebebereich zwischen den Rippen und einem an die Rippen angrenzenden Gewebebereich entsprechen.

15. Nichtflüchtiges computerlesbares Medium, auf dem Anweisungen zum Veranlassen einer Verarbeitungsschaltung gespeichert sind, einen Prozess auszuführen, wobei der Prozess umfasst:
Empfangen von Daten, die für ein dreidimensionales Diagnosebild repräsentativ sind, wobei das dreidimensionale Diagnosebild Rippen und Wirbelsäule eines Subjekts einschließt;
Segmentieren der Rippen und Wirbelsäule gemäß den empfangenen Daten, die repräsentativ für das dreidimensionale Diagnosebild sind;
Erkennen und Beschriften von Rippenmittellinien aus der Rippensegmentierung;
Erkennen und Beschriften von Wirbelkörpermittelpunkt-Orientierungspunkten aus der Wirbelsäulensegmentierung;
Definieren einer zweidimensionalen Mannigfaltigkeitsebene zur Bezeichnung einer Visualisierungsfläche zum Anzeigen eines neu formatierten Bildes, wobei die zweidimensionale Mannigfaltigkeitsebene verformt wird, um sich mit den erkannten Rippenmittellinien und Wirbelmittelpunkt-Orientierungspunkten auszurichten;
Abbilden jeder dreidimensionalen Position, die den empfangenen Daten entspricht, die für das dreidimensionale Diagnosebild repräsentativ sind, der Rippenmittellinien und der Wirbelmittelpunkt-Orientierungspunkte auf eine zweidimensionale Position auf der definierten zweidimensionalen Ebene;
Interpolieren dreidimensionaler Positionskoordinaten, die auf der definierten zweidimensionalen Mannigfaltigkeitsebene fehlen, wodurch die zweidimensionale Mannigfaltigkeit so verformt wird, dass die zweidimensionale Mannigfaltigkeit mit den erkannten Rippenmittellinien und Wirbelmittelpunkt-Orientierungspunkten ausgerichtet ist;
Abtasten von Bildintensitäten aus einem dreidimensionalen Diagnosebildraum an jeder Koordinate der verformten zweidimensionalen Mannigfaltigkeitsebene;
Generieren des neu formatierten Bildes als Mannigfaltigkeitsschnitt aus den abgetasteten Bildintensitäten, wobei der Mannigfaltigkeitsschnitt eine kontinuierliche und begradigte Visualisierung eines Brustkorbs und einer Wirbelsäule anzeigt;
Verschieben der verformten zweidimensionalen Mannigfaltigkeitsebene entlang ihrer Normalenrichtungen und Wiederholen der Abtastung, um einen Stapel von Mannigfaltigkeitsschnitten zu generieren, die eine vollständige dreidimensionale Visualisierung des Brustkorbs abdecken;
Generieren einer dreidimensionalen Visualisierung des Brustkorbs und der Wirbelkörpermittelpunkte als einen Stapel von Mannigfaltigkeiten, die den gesamten Brustkorb abdecken.

## Revendications

1. Dispositif de traitement d'image, comprenant :
une mémoire configurée pour stocker des instructions exécutables par ordinateur ; et
au moins un processeur configuré pour exécuter les instructions exécutables par ordinateur afin d'amener le dispositif de traitement d'image à :
recevoir (402) des données représentatives d'une image de diagnostic tridimensionnelle, l'image de diagnostic tridimensionnelle incluant les côtes et la colonne vertébrale d'un sujet ;
segmenter (404) les côtes et la colonne vertébrale selon les données reçues représentatives de l'image de diagnostic tridimensionnelle ;
détecter et étiqueter (406) des axes médians de côte à partir de la segmentation de côtes ;
détecter et étiqueter (408) des points de repère de centre de corps de vertèbre à partir de la segmentation de colonne vertébrale ;
définir (410) un plan de variété bidimensionnelle pour signifier un canevas de visualisation pour afficher une image reformatée ;
mapper (412) chaque position tridimensionnelle, correspondant aux données reçues représentatives de l'image de diagnostic tridimensionnelle, des axes médians de côte et des points de repère de centre de vertèbre sur une position bidimensionnelle sur le plan bidimensionnel défini ;
interpoler (414) des coordonnées de position tridimensionnelles manquantes sur le plan de variété bidimensionnelle défini, ce qui déforme la variété bidimensionnelle de sorte que la variété bidimensionnelle s'aligne sur les axes médians de côte et les points de repère de centre de vertèbre détectés ;
échantillonner (416) des intensités d'image, à partir d'un espace d'image de diagnostic tridimensionnelle, au niveau de chaque coordonnée du plan de variété bidimensionnelle déformé ;
générer (418) l'image reformatée sous forme d'une tranche de variété à partir des intensités d'image échantillonnées, la tranche de variété affichant une visualisation continue et redressée d'une cage thoracique et d'une colonne vertébrale ;
décaler (420) le plan de variété bidimensionnelle déformé le long de ses directions normales et répéter l'échantillonnage pour générer (422) une pile de tranches de variété couvrant une visualisation tridimensionnelle complète de la cage thoracique et de la colonne vertébrale ; et
générer (424) une visualisation tridimensionnelle de la cage thoracique et des centres de corps de vertèbre sous la forme d'une pile de variétés couvrant toute la cage thoracique.

2. Dispositif de traitement d'image selon la revendication 1, dans lequel la segmentation des côtes et de la colonne vertébrale est effectuée avec des techniques d'apprentissage automatique ou d'apprentissage profond, les techniques d'apprentissage automatique ou d'apprentissage profond incluant au moins un certain parmi des réseaux neuronaux, une régression logistique, des forêts aléatoires, les plus proches voisins et une analyse par grappes ou multivariée.

3. Dispositif de traitement d'image selon la revendication 1, dans lequel l'interpolation est effectuée avec des techniques de spline à plaque mince.

4. Dispositif de traitement d'image selon la revendication 1, dans lequel le processeur est en outre configuré pour exécuter les instructions exécutables par ordinateur pour amener le dispositif de traitement d'image à calculer les directions normales pour chaque point sur le plan bidimensionnel.

5. Dispositif de traitement d'image selon la revendication 1, dans lequel la pile générée de tranches de variété est une pile de reconstructions multiplanaires bidimensionnelles interpolées.

6. Dispositif de traitement d'image selon la revendication 5, dans lequel le processeur est en outre configuré pour exécuter les instructions exécutables par ordinateur afin de générer une image tridimensionnelle de la cage thoracique et de la colonne vertébrale sur la base de la pile de reconstructions multiplanaires bidimensionnelles.

7. Dispositif de traitement d'image selon la revendication 1, dans lequel les intensités d'image échantillonnées à chaque position du plan de variété bidimensionnelle déformé correspondent à au moins l'une d'une région de tissu entre les côtes et d'une région de tissu bordant les côtes.

8. Procédé mis en œuvre par ordinateur de traitement d'une image tridimensionnelle, le procédé comprenant :
la réception (402) de données représentatives d'une image de diagnostic tridimensionnelle, l'image de diagnostic tridimensionnelle incluant les côtes et la colonne vertébrale d'un sujet ;
la segmentation (404) des côtes et de la colonne vertébrale selon les données reçues représentatives de l'image de diagnostic tridimensionnelle ;
la détection et l'étiquetage (406) d'axes médians de côte à partir de la segmentation de côtes ;
la détection et l'étiquetage (408) de points de repère de centre de corps de vertèbre à partir de la segmentation de colonne vertébrale ;
la définition (410) d'un plan de variété bidimensionnelle pour signifier un canevas de visualisation pour afficher une image reformatée ;
le mappage (412) de chaque position tridimensionnelle, correspondant aux données reçues représentatives de l'image de diagnostic tridimensionnelle, des axes médians de côte et des points de repère de centre de vertèbre sur une position bidimensionnelle sur le plan bidimensionnel défini ;
l'interpolation (414) de coordonnées de position tridimensionnelles manquantes sur le plan de variété bidimensionnelle défini, ce qui déforme la variété bidimensionnelle de sorte que la variété bidimensionnelle s'aligne sur les axes médians de côte et les points de repère de centre de vertèbre détectés ;
l'échantillonnage (416) d'intensités d'image, à partir d'un espace d'image de diagnostic tridimensionnelle, au niveau de chaque coordonnée du plan de variété bidimensionnelle déformé ;
la génération (418) de l'image reformatée sous forme d'une tranche de variété à partir des intensités d'image échantillonnées, la tranche de variété affichant une visualisation continue et redressée d'une cage thoracique et d'une colonne vertébrale ;
le décalage (420) du plan de variété bidimensionnelle déformé le long de ses directions normales et la répétition de l'échantillonnage pour générer (422) une pile de tranches de variété couvrant une visualisation tridimensionnelle complète de la cage thoracique et de la colonne vertébrale ;
la génération (424) d'une visualisation tridimensionnelle de la cage thoracique et des centres de corps de vertèbre sous la forme d'une pile de variétés couvrant toute la cage thoracique.

9. Procédé selon la revendication 8, dans lequel la segmentation est effectuée avec des techniques d'apprentissage automatique ou d'apprentissage profond, les techniques d'apprentissage automatique ou d'apprentissage profond incluant au moins un certain parmi des réseaux neuronaux, une régression logistique, des forêts aléatoires, les plus proches voisins et une analyse par grappes ou multivariée.

10. Procédé selon la revendication 8, dans lequel l'interpolation est effectuée avec des techniques de spline à plaque mince.

11. Procédé selon la revendication 8, comprenant en outre le calcul des directions normales pour chaque point sur le plan bidimensionnel.

12. Procédé selon la revendication 8, dans lequel la pile générée de tranches de variété est une pile de reconstructions multiplanaires bidimensionnelles interpolées.

13. Procédé selon la revendication 12, comprenant en outre la génération d'une image tridimensionnelle de la cage thoracique et de la colonne vertébrale sur la base de la pile de reconstructions multiplanaires bidimensionnelles interpolées.

14. Procédé selon la revendication 8, dans lequel les intensités d'image échantillonnées à chaque position du plan de variété bidimensionnelle déformé correspondent à au moins l'une d'une région de tissu entre les côtes et d'une région de tissu bordant les côtes.

15. Support non transitoire lisible par ordinateur sur lequel sont stockées des instructions amenant une circuiterie de traitement à exécuter un processus, le processus comprenant :
la réception de données représentatives d'une image de diagnostic tridimensionnelle, l'image de diagnostic tridimensionnelle incluant les côtes et la colonne vertébrale d'un sujet ;
la segmentation des côtes et de la colonne vertébrale selon les données reçues représentatives de l'image de diagnostic tridimensionnelle ;
la détection et l'étiquetage d'axes médians de côte à partir de la segmentation de côtes ;
la détection et l'étiquetage de points de repère de centre de corps de vertèbre à partir de la segmentation de colonne vertébrale ;
la définition d'un plan de variété bidimensionnelle pour signifier un canevas de visualisation afin d'afficher une image reformatée, le plan de variété bidimensionnelle étant déformé pour s'aligner sur les axes médians de côtes et les repères de centre de corps de vertèbres détectés ;
le mappage de chaque position tridimensionnelle, correspondant aux données reçues représentatives de l'image de diagnostic tridimensionnelle, des axes médians de côte et des points de repère de centre de vertèbre sur une position bidimensionnelle sur le plan bidimensionnel défini ;
l'interpolation de coordonnées de position tridimensionnelles manquantes sur le plan de variété bidimensionnelle défini, ce qui déforme la variété bidimensionnelle de sorte que la variété bidimensionnelle s'aligne sur les axes médians de côte et les points de repère de centre de vertèbre détectés ;
l'échantillonnage d'intensités d'image, à partir d'un espace d'image de diagnostic tridimensionnelle, au niveau de chaque coordonnée du plan de variété bidimensionnelle déformé ;
la génération de l'image reformatée sous forme d'une tranche de variété à partir des intensités d'image échantillonnées, la tranche de variété affichant une visualisation continue et redressée d'une cage thoracique et d'une colonne vertébrale ;
le décalage du plan de variété bidimensionnelle déformé le long de ses directions normales et la répétition de l'échantillonnage pour générer une pile de tranches de variété couvrant une visualisation tridimensionnelle complète de la cage thoracique ; et
la génération d'une visualisation tridimensionnelle de la cage thoracique et des centres de corps de vertèbre sous la forme d'une pile de variétés couvrant toute la cage thoracique.
